(19) Europäisches Patentamt
European Patent Office
Office européen des brevets

(11) Publication number: 0 338 650
A1

# (12) EUROPEAN PATENT APPLICATION

(21) Application number: 89201014.1

(51) Int. Cl.4: C07D 403/06 , A61K 31/415

(22) Date of filing: 20.04.89

(30) Priority: 22.04.88 NL 8801047

(43) Date of publication of application:
25.10.89 Bulletin 89/43

(84) Designated Contracting States:
AT BE CH DE ES FR GB GR IT LI LU NL SE

(71) Applicant: DUPHAR INTERNATIONAL RESEARCH B.V
C.J. van Houtenlaan 36
NL-1381 CP Weesp(NL)

(72) Inventor: Haeck, Hans H.
c/o OCTROOIBUREAU ZOAN B.V. P.O. Box 140
NL-1380 AC Weesp(NL)

Inventor: Hamminga, Derk
c/o OCTROOIBUREAU ZOAN B.V. P.O. Box 140
NL-1380 AC Weesp(NL)
Inventor: van Wijngaarden, Ineke
c/o OCTROOIBUREAU ZOAN B.V. P.O. Box 140
NL-1380 AC Weesp(NL)
Inventor: Wouters, Wouter
c/o OCTROOIBUREAU ZOAN B.V. P.O. Box 140
NL-1380 AC Weesp(NL)

(74) Representative: Muis, Maarten et al
OCTROOIBUREAU ZOAN B.V. P.O. Box 140
NL-1380 AC Weesp(NL)

## (54) New imidazolylmethyl-cycloalkane(b)indolones.

(57) The invention relates to new imidazolylmethyl-cycloalkane[b]indolones having formula

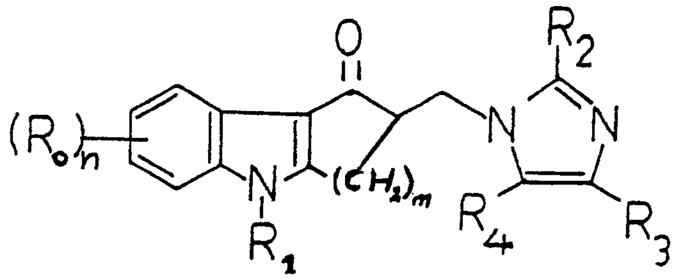

(2)

wherein
- $R_0$ is alkyl or alkoxy having 1-4 C-atoms, hydroxy, halogen, trifluoromethyl, a group $R_6R_7N$ or $R_6R_7$-N-CO, wherein $R_6$ and $R_7$ are hydrogen or alkyl having 1-4 C-atoms or wherein $R_6R_7N$ is a saturated 5-6 ring and n has the value 0, 1 or 2,
- $R_1$ is hydrogen, 1-8 C alkyl, 3-7 C cycloalkyl, 3-7 C cycloalkyl-(1-3 C)- alkyl, 3-6 C alkenyl, 3-8 C alkynyl, phenyl or phenyl-(1-3 C) alkyl, wherein the phenyl group may be substituted with one or more alkyl groups, alkoxy groups or OH-groups or with one or more halogen atoms, or $R_1$ is a group $COOR_8$, $COR_8$, $SO_2R_8$, wherein $R_8$ is 1-4 C alkyl, 3-7 C cycloalkyl, phenyl or phenyl-(1-2 C)alkyl wherein the phenyl group may be substituted with one or more 1-4 C alkyl groups, 1-4 C alkoxy groups, hydroxyl groups or halogen atoms, or $R_1$ is a group $R_9R_{10}$N-C $^0$ wherein $R_9$ and $R_{10}$ independently of each other are hydrogen, 1-4 C alkyl, phenyl or phenyl-(1-2 C)alkyl wherein the phenyl group may be substituted, or wherein $R_9$ and $R_{10}$ together

EP 0 338 650 A1

with the nitrogen atom to which they are bound constitute a saturated 5-6 ring,
- one of the groups $R_2$, $R_3$ and $R_4$ is hydrogen, alkyl having 1-4 C atoms, cycloalkyl having 3-6 C-atoms, alkenyl having 2-4 C-atoms or phenyl-alkyl having 1-2 C-atoms in the alkyl group and the two other groups, independently of each other, are hydrogen or alkyl having 1-4 C-atoms,
- m has the value 3 to 8 inclusive,

and the pharmaceutically acceptable acid addition salts thereof.

The compounds have a longer-lasting selective antagonistic activity on "neuronal" 5-hydroxytryptamine (5-HT) receptors as compared with related known compounds, and they are less toxic.

# New imidazolylmethyl-cycloalkane[b]indolones

The invention relates to a group of new cycloalkane[b]indolones which comprise at the carbon atom, besides the carbonyl group, an imidazolylmethyl group as a substituent, to the preparation thereof, and to compositions which comprise at least one of these compounds as an active substance.

It is known from Belgian Patent Specification no. 901576 and European Patent Application no. 86305671.9 (publication no. 0210840) that carbazolone compounds of formula 1

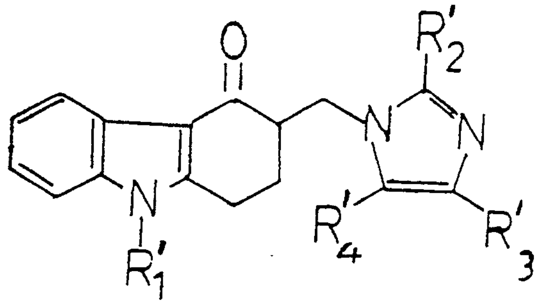

(1)

wherein $R_1'$ is hydrogen, alkyl having 1-10 C-atoms, cycloalkyl having 3-7 C-atoms, alkenyl having 3-6 C-atoms, phenyl or phenylalkyl (1-3 C in the alkyl group) and a group $CO_2R_5'$, $COR_5'$, $CONR_5'R_6'$ or $SO_2R_5'$, respectively, (wherein $R_5'$ and $R_6'$ may inter alia be alkyl or cycloalkyl) and wherein one of the groups $R_2'$, $R_3'$ and $R_4'$ is hydrogen, alkyl (1-6 C) cycloalkyl (3-7 C), alkenyl (2-6 C) or phenylalkyl (1-3 C in the alkyl group), and the two other groups may be hydrogen or alkyl (1-6 C), are strong and selective antagonists of "neuronal" 5-hydroxytryptamine (5-HT) receptors.

It has been found surprisingly that compounds of formula 2

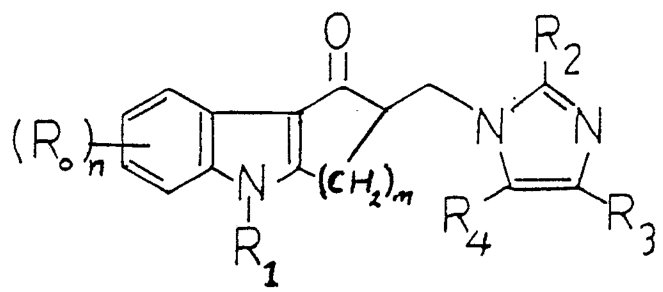

(2)

wherein

- $R_0$ is alkyl or alkoxy having 1-4 C-atoms, hydroxy, halogen, trifluoromethyl, a group $R_6R_7N$ or $R_6R_7$-N-CO, wherein $R_6$ and $R_7$ are hydrogen or alkyl having 1-4 C-atoms or wherein $R_6R_7N$ is a saturated 5-6 ring and n has the value 0, 1 or 2,
- $R_1$ is hydrogen, 1-8 C alkyl, 3-7 C cycloalkyl, 3-7 C cycloalkyl-(1-3 C)- alkyl, 3-6 C alkenyl, 3-8 C alkynyl, phenyl or phenyl-(1-3 C) alkyl, wherein the phenyl group may be substituted with one or more alkyl groups, alkoxy groups or OH-groups or with one or more halogen atoms, or R1 is a group $COOR_8$, $COR_8$, $SO_2R_8$, wherein $R_8$ is 1-4 C alkyl, 3-7 C cycloalkyl, phenyl or phenyl-(1-2 C)alkyl wherein the phenyl group may be substituted with one or more 1-4 C alkyl groups, 1-4 C alkoxy groups, hydroxyl groups or halogen atoms, or $R_1$ is a group

$$R_9R_{10}N-C{=}O$$

wherein $R_9$ and $R_{10}$ independently of each other are hydrogen, 1-4 C alkyl, phenyl or phenyl-(1-2 C)alkyl wherein the phenyl group may be substituted, or wherein $R_9$ and $R_{10}$ together with the nitrogen atom to which they are bound constitute a saturated 5-6 ring,
- one of the groups $R_2$, $R_3$ and $R_4$ is hydrogen, alkyl having 1-4 C atoms, cycloalkyl having 3-6 C-atoms, alkenyl having 2-4 C-atoms or phenyl-alkyl having 1-2 C-atoms in the alkyl group and the two other groups,

independently of each other, are hydrogen or alkyl having 1-4 C-atoms,
- m has the value 3 to 8 inclusive,
and the pharmaceutically acceptable acid addition salts thereof have a similar but considerably longer-lasting activity and a lower toxicity than the known compounds of formula 1.

Examples of suitable acids with which the compounds of formula 2 according to the invention can form pharmaceutically acceptable acid addition salts are hydrochloric acid, sulphuric acid, phosphoric acid, nitric acid, and organic acids, for example, citric acid, fumaric acid, maleic acid, tartaric acid, acetic acid, benzoic acid, p-toluenesulphonic acid, methane sulphonic acid and the like.

The carbon atom to which the imidazolyl methyl group is bound is a centre of chirality. Both the racemates and the individual enantiomers of compounds of formula 2 belong to the invention.

The antagonistic activity of the compounds of formula 2 on the response induced by 5-HT was determined and measured in the Bezold-Jarisch reflex test in rats.

The affinity for "neuronal" 5-HT receptors was determined and measured by means of the displacement of ($^3$H)GR 38032F of neuroblastoma cells.

On the basis of the antagonistic activity of this type of 5-HT receptors, the compounds may be used for the treatment of symptoms which are caused by over-excitation of these receptors a) in the gastroitestinal system (nausea and vomitting as a result of exogenic factors, for example, cancer therapy, or endogenic factors, for example, stasis of the stomach and migraine), ulcer, dyspepsia, spasms, irritable bowel syndrome, etc., or b) in the central nervous system (hallucinations, delusions, depressions, manias, fear, pain, improvement of vigilance or mood etc.), or c) in the cardiovascular system, for example, spasms of the vessels, arrhytmia, etc., or d) in the respiratory system (including nasal disorders or disorders of the bronchi and lungs), or e) for the relief or prevention of withdrawal syndromes induced by abuse of drugs.

The compounds according to the invention and their salts can be brought into a form suitable for administration, for example, pills, tablets, coated tablets, capsules, powders, injection liquids and the like by means of techniques conventionally used for this purpose and while using suitable auxilairy substances, for example, solid or liquid carrier materials.

The dosage in which the compounds according to the invention may be used depends on the severity and the nature of the disease to be treated and on the way of administration. As a rule the dosage will be between 0.05 and 20 mg, preferably between 0.1 and 10 mg of active substance daily.

The compounds according to the invention may be prepared in a manner known for analogous compounds. Suitable methods of preparing this type of compounds are described, for example, in the above-mentioned European Patent Application published under no.0210840.

In particular, the compounds of formula 2 can be obtained in a good yield by
(a) reaction of a compound of formula 3

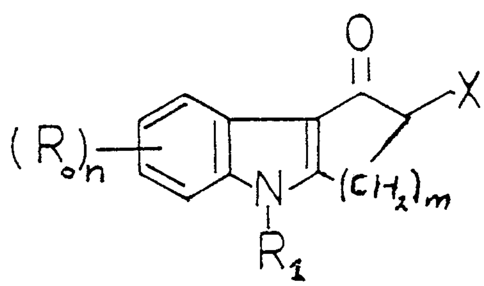

(3)

wherein $R_0$, n, m and $R_1$ have the meanings mentioned hereinbefore, and X is a reactive group, preferably the group $=CH_2$ or $-CH_2N(CH_3)_2$, with an imidazole compound of formula 4

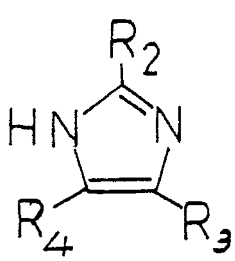

(4)

or a salt thereof, wherein $R_2$, $R_3$ and $R_4$ have the meanings mentioned hereinbefore.

The reaction is preferably carried out in a suitable solvent, for example, water, alcohol, dimethylformamide, etc., at temperatures between 20°C and 150°C.

The starting compounds of formula 3 to be used in this reaction may be obtained, for example, by reaction of a compound of formula 5

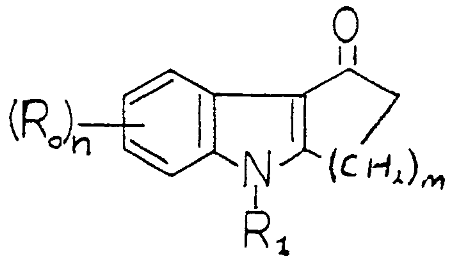

(5)

wherein $R_0$, n, m, and $R_1$ have the above-mentioned meanings, with formaldehyde and dimethylamine hydrochloride, preferably in an organic solvent, for example, acetic acid or alcohol, while heating.

The starting substances of formula 5 are known compounds or they can be obtained in a manner known per se, for example, by 1) ring closure of compounds of formula 6

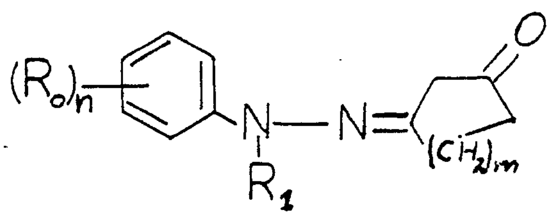

(6)

wherein $R_0$, n, m and $R_1$ have the above-mentioned meanings. This ring closure reaction may be carried out by boiling in a organic solvent, for example, acetic acid in the presence of an acid catalyst, for example, concentrated hydrochloric acid or sulphuric acid.

The compounds of formula 6 are known compounds or they may be obtained analogously to known compounds.

2) by oxidation of a compound of formula 7

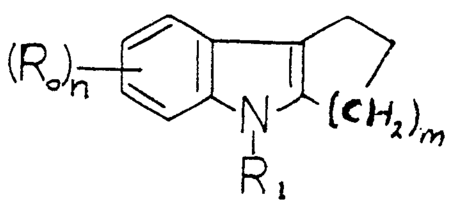

(7)

wherein $R_0$, n, m and $R_1$ have the above-mentioned meanings, with a suitable oxidant, for example, 2,3-dichloro-5,6-dicyano-1,4-benzoquinone (DDQ) or selenium dioxide, preferably in a suitable solvent, for example, water, tetrahydrofuran or dioxan. In particular, the starting substances of formula 5 can be obtained in a good yield by oxidation with DDQ of the analogous compounds of formula 7 in tetrahydrofuran and water at temperatures between -10 and 20°C, for example, as described for similar compounds in J.Org.Chem. 42, (1977), 1213.

3) by reaction of a compound of formula 5 wherein $R_1$ is a hydrogen atom or a metal atom, with a compound of formula 8

$R_1$ - Y (8)

wherein $R_1$ has the meaning given in formula 2, with the proviso that $R_1$ is not a hydrogen atom, and wherein Y is a reactive group, for example, a halogen atom or a $-OSO_2-O$-alkyl group. The reaction is preferably carried out in a solvent, for example, dimethyl formamide, acetone, alcohol, an arylhalogenide etc. at temperatures between 0 and 250°C, whether or not in the presence of a base for example, potassium hydroxide, potassium carbonate etc., in the presence or absence of a catalyst such as copper,

copper oxide, copper-(I)-halogenide, copper oxide on silica gel, etc.

(b) Compounds of formula 2 can also be obtained by reaction of a compound of formula 2 wherein $R_1$ is a hydrogen atom or a metal atom and $R_0$, n, m, $R_2$, $R_3$ and $R_4$ have the meanings mentioned in formula 2, with a compound of formula 8, wherein $R_1$ has the meaning mentioned in formula 8. The reaction is preferably carried out in a solvent, for example, acetone, alcohol, DMF or dioxan, an arylhalogenide, etc. at temperatures between 0 and 250° C, whether or not in the presence of a base, for example, potassium hydroxide potasssium carbonate etc., in the presence or absence of a catalyst such as copper, copper-(I)-halogenide, etc.

The invention will now be described in greater detail with reference to the ensuing specific examples.

EXAMPLE I

6,7,8,9-tetrahydro-5-methyl-9-[(2-methyl-1H-imidazol-1-yl)-methyl]cyclohept[b]indol-10(5H)one

(a) A mixture of 6.8 g (34 mmol) of 6,7,8,9-tetrahydrocyclohept[b]-indol-10(5H)-one (prepared according to J.Org.Chem. 42 (1977) 1213, 2.0 g (68 mmol) of paraformaldehyde, 6.1 g (75 mmol) of dimethylamine hydrochloride and 100 ml of acetic acid was heated at 100° C for 2 hours while stirring. The reaction mixture was then evaporated to dryness and dissolved in 100 ml of water. This solution was rendered alkaline with 2N sodium hydroxide solution and extracted with dichloromethane. This extract was washed with water, dried and evaporated to dryness. The resulting crude product was purified by chromatography over 300 g of silicagel, elution being carried out with methanoltriethylamine (97 : 3). The fraction with rf 0.4 gave 6.4 g of crude product. This was dissolved in 50 ml of ethanol to which 2.5 ml of concentrated hydrochloric acid were added. The product was then fully evaporated to dryness. The residue was dissolved in 20 ml of boiling absolute ethanol after which 20 ml of ethyl acetate were added. After leaving to stand overnight at 0° C the crystallised substance was sucked off. Yield 5.6 g of 6,7,8,9-tetrahydro-9-dimethylaminomethyl-cyclohept[b]indol-10(5H)-one hydrochloride having a melting-point of 152°-154° C (with gas evolution).

(b) 4.4 g (15 mmol) of the hydrochloride obtained according to a), 3.7 g (45 mmol) of 2-methylimidazole, 45 ml of water and 25 ml of 1-propanol were boiled while stirring for 24 hours. The mixture was then cooled while stirring, the desired compound crystallising. 50 Ml of water were added to the mixture and the mixture was cooled to 0 ° C. The crystallised substance was sucked off, washed with water and dried. Yield 3.8 g of 6,7,8,9-tetrahydro-9-[(2-methyl-1H-imidazol-1-yl)methyl]-cyclohept[b]indol-10-(5H)-one having a melting-point of 185-186° C.

(c) 1.8 g (30 mmol) of powdered potassium hydroxide were added in one portion to 2.7 g (9.2 mmol) of the compound obtained according to b) in 50 ml of acetone while stirring. After stirring for 10 minutes the mixture was poured out on 200 ml of ice water. This was extracted with dichloromethane The extract was evaporated to dryness. The residue was purified by chromatography over 150 g of silica gel, elution being carried out with dichloromethane -methanol (95 : 5). The fraction with rf 0.25 was isolated. Yield 1.9 g of the desired product having a melting-point of 173-175° C.

In an analogous manner the following compound has been obtained:

6,7,8,9-tetrahydro-4-chloro-5-methyl-9-[(2-methyl-1H-imidazol-1-yl)-methyl]-cyclohept[b]indol-10(5H)-one.

EXAMPLE II

6,7,8,9-Tetrahydro-5-cyclohexyl-9-[2-methyl-1H-imidazol-1-yl)-methyl]-cyclohept[b]indol-10(5H)-one hydrochloride

(a) A mixture of 5.8 g (20.6 mmol) of 6,7,8,9-tetrahydro-5-cyclohexyl-cyclohept[b]indol-10(5H)-one (obtained by oxidation of 6,7,8,9-tetrahydro-5-cyclohexyl-5H,10H-cyclohept[b]indole according to J. Org. Chem. 42, (1977), page 1213), 1.2 g (41.3 mmol)of paraformaldehyde, 3.7 g (45.4 mmol) of dimethylamine hydrochloride, and 60 ml of acetic acid were heated at 100° C for 90 minutes while stirring. Then the reaction mixture was cooled and made alkaline with a solution of potassium carbonate. The alkaline mixture was shaken with methylene chloride and evaporated. The residue was chromatographed over silica gel by eluting with methylene chloride/methanol/ammonia (92.5:7:0.5). Fractions containing the desired product

were evaporated, the residue was dissolved in ethyl acetate, and an excess of alcoholic hydrochloric acid was added. The salt was sucked off and dried. In this manner 5.4 g (70%) of 6,7,8,9-tetrahydro-5-cyclohexyl-9-(dimethylamino)methyl-cyclohept[b]indol-10(5H)-one hydrochloride were obtained.

(b) A mixture of 4.5 g of the obtained product, 3.0 g (36 mmol) of 2-methyl-imidazole, 36 ml of water and 20 ml of 1-propanol was boiled for 24 hours. After cooling 2N sodium hydroxide solution was added, and the mixture was shaken with methylene chloride. The methylene chloride layer was washed with water and evaporated under reduced pressure. The residue was chromatographed over silica gel with methylene chloride/methanol/ammonia (92.5:7:0.5) as an eluent. The desired fractions were evaporated, the residue was dissolved in ethyl acetate, and an excess of alcoholic hydrochloric acid was added. The solid substance was sucked off and dried. In this manner 3.2 g (65%)of the desired hydrochloride having a melting point of 45 -50 °C were obtained.

In an analogous manner the following compounds were obtained:
6,7,8,9-tetrahydro-5-cyclopropylmethyl-9-[(2-methyl-1H-imidazol-1-yl)-methyl]-cyclohept[b]indol-10(5H)-one hydrochloride, melting point 213-215 °C; and
6,7,8,9,10,11-hexahydro-5-methyl-10- [(2-methyl-1H-imidazol-1-yl)methyl]-5H-cyclooct[b]indol-11-one, melting point 178-180 °C.

EXAMPLE III

6,7,8,9-Tetrahydro-5-(4-fluorophenyl)-9-[(2-methyl-1H-imidazol-1-yl)-methyl]-cyclohept[b]indol-10(5H)-one hydrochloride

According to the method described in Synthesis (1985) pages 856-860 (Goldberg reaction) 6,7,8,9-tetrahydrocyclohept[b]indol-10(5H)-one was reacted with 1-bromo-4- fluorobenzene. According to the method as described in Example II 1.0 g (58%) of the desired hydrochloride (melting point 253-255 °C) was obtained from 1.2 g (4.1 mmol) of the obtained 6,7,8,9-tetrahydro-5-(4-fluorophenyl)-cyclohept[b]indol-10-(5H)-one.

## Claims

1. Compounds of formula 2

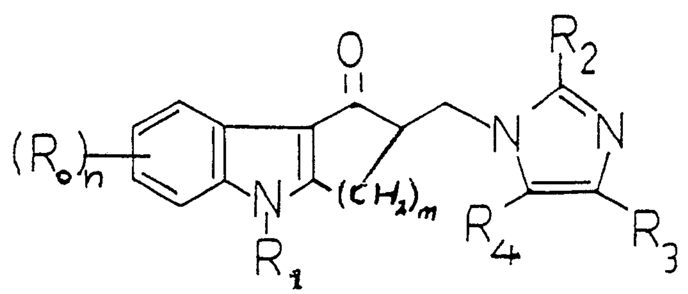

(2)

wherein
- $R_0$ is alkyl or alkoxy having 1-4 C-atoms, hydroxy, halogen, trifluoromethyl, a group $R_6R_7N$ or $R_6R_7N$-CO, wherein $R_6$ and $R_7$ are hydrogen or alkyl having 1-4 C-atoms or wherein $R_6R_7N$ is a saturated 5-6 ring and n has the value 0, 1 or 2,
- $R_1$ is hydrogen, 1-8 C alkyl, 3-7 C cycloalkyl, 3-7 C cycloalkyl-(1-3 C)- alkyl, 3-6 C alkenyl, 3-8 C alkynyl, phenyl or phenyl-(1-3 C) alkyl, wherein the phenyl group may be substituted with one or more alkyl groups, alkoxy groups or OH-groups or with one or more halogen atoms, or $R_1$ is a group $COOR_8$, $COR_8$, $SO_2R_8$, wherein $R_8$ is 1-4 C alkyl, 3-7 C cycloalkyl, phenyl or phenyl-(1-2 C)alkyl wherein the phenyl group may be substituted with one or more 1-4 C alkyl groups, 1-4 C alkoxy groups, hydroxyl groups or halogen atoms, or $R_1$ is a group

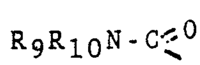

wherein $R_9$ and $R_{10}$ independently of each other are hydrogen, 1-4 C alkyl, phenyl or phenyl-(1-2 C)alkyl wherein the phenyl group may be substituted, or wherein $R_9$ and $R_{10}$ together with the nitrogen atom to which they are bound constitute a saturated 5-6 ring,

- one of the groups $R_2$, $R_3$ and $R_4$ is hydrogen, alkyl having 1-4 C atoms, cycloalkyl having 3-6 C-atoms, alkenyl having 2-4 C-atoms or phenyl-alkyl having 1-2 C-atoms in the alkyl group and the two other groups, independently of each other, are hydrogen or alkyl having 1-4 C-atoms, - m has the value 3 to 8 inclusive, and the pharmaceutically acceptable acid addition salts thereof.

2. Pharmaceutical compositions which comprise at least one compound as claimed in Claim 1 as an active substance.

3. A method of preparing pharmaceutical compositions as claimed in Claim 2, characterised in that a compound of formula 2 wherein the symbols have the meaning mentioned in Claim 1, or a pharmaceutically acceptable acid addition salt thereof is brought into a form suitable for administration.

4. A method of preparing compounds as claimed in Claim 1, characterised in that compounds of formula 2 wherein the symbols have the meanings mentioned in Claim 1 are prepared in a manner known for the synthesis of analogous compounds.

5. A method as claimed in Claim 4, characterised in that a compound of formula 3

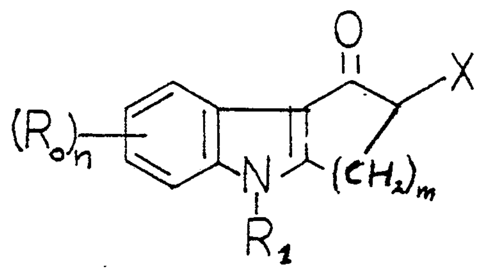

(3)

wherein in $R_0$, n, m and $R_1$ have the meanings mentioned in Claim 1, and X is a reactive group, is converted with a compound of formula 4

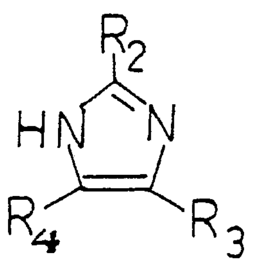

(4)

wherein $R_2$, $R_3$, and $R_4$ have the meanings mentioned in Claim 1.

6. A method as claimed in Claim 5, characterised in that as a starting substance of formula 3 a compound is used in which X is the group $=CH_2$ or $-CH_2N(CH_3)_2$.

7. A method as claimed in Claim 4, characterised in that a compound of formula 2 wherein $R_0$, n, m, $R_2$, $R_3$ and $R_4$ have the meanings mentioned in Claim 1 and $R_1$ is hydrogen or a metal atom, is converted with a compound of the formula $R_1$-Y, wherein $R_1$ has one of the remaining meanings given in claim 1, and Y is a reactive group.

8. A method as claimed in Claim 7, characterised in that Y is a halogen atom or a group $-OSO_2-O-$alkyl, wherein alkyl is an alkyl group having 1-4 C-atoms.

European Patent Office

# EUROPEAN SEARCH REPORT

Application Number

EP 89 20 1014

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4 ) |
| D,A | EP-A-210840 (GLAXO GROUP LIMITED) --- | | C07D403/06<br>A61K31/415 |
| D,A | GB-A-2153821 (GLAXO GROUP LIMITED) --- | | |
| P,A | DE-A-3740352 (GLAXO GROUP LIMITED) ----- | | |
| | | | TECHNICAL FIELDS SEARCHED (Int. Cl.4 ) |
| | | | C07D403/00<br>A61K31/00 |

The present search report has been drawn up for all claims

| Place of search | Date of completion of the search | Examiner |
|---|---|---|
| THE HAGUE | 17 JULY 1989 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document